(19) Europäisches Patentamt
European Patent Office
Office européen des brevets

(11) EP 3 072 499 B1

(12) **EUROPÄISCHE PATENTSCHRIFT**

(45) Veröffentlichungstag und Bekanntmachung des Hinweises auf die Patenterteilung:
**17.07.2019 Patentblatt 2019/29**

(51) Int Cl.:
***A61K 8/35*** *(2006.01)* ***A61K 8/49*** *(2006.01)*
***A61Q 17/04*** *(2006.01)* ***A61K 8/34*** *(2006.01)*

(21) Anmeldenummer: **16163784.8**

(22) Anmeldetag: **22.01.2015**

(54) **OCTOCRYLENFREIES SONNENSCHUTZMITTEL MIT GERINGER KLEBRIGKEIT**

OCTOCRYLENE-FREE SUNSCREEN WITH LOW ADHESION

PRODUIT DE PROTECTION CONTRE LE SOLEIL SANS OCTOCRILENE A FAIBLE ADHERENCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.01.2014 DE 102014201541**

(43) Veröffentlichungstag der Anmeldung:
**28.09.2016 Patentblatt 2016/39**

(62) Dokumentnummer(n) der früheren Anmeldung(en) nach Art. 76 EPÜ:
**15152074.9 / 2 905 012**

(73) Patentinhaber: **Beiersdorf AG**
**20253 Hamburg (DE)**

(72) Erfinder:
- **Schade, Tatjana**
  **25866 Mildstedt/Rosendahl (DE)**
- **Skubsch, Kerstin**
  **25497 Prisdorf (DE)**
- **Brinkmann, Sina**
  **21698 Harsefeld (DE)**
- **Bleckmann, Andreas**
  **22926 Ahrensburg (DE)**
- **Schlenker, David**
  **22607 Hamburg (DE)**

(56) Entgegenhaltungen:
**DE-A1- 19 846 771** **DE-A1-102012 200 074**
**US-A1- 2013 344 015**

- **DATABASE GNPD [Online] MINTEL; 1 July 2012 (2012-07-01), Migros: "Protect & Tan Sun Milk SPF 20 Medium", Database accession no. 1826191**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 072 499 B1

## Beschreibung

Die vorliegende Erfindung betrifft eine kosmetische Zubereitung enthaltend eine UV-Filterkombination aus 4-(tert.-Butyl)-4'-methoxydibenzoylmethan, 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) und 2-Phenylbenzimidazol-5-sulfonsäuresalzen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Sonnenschutzmittel weisen unter anderem aufgrund ihres UV-Filtergehaltes meist eine gewisse Klebrigkeit auf, die insbesondere bei der Verwendung am Strand dazu führt, dass es auf eingecremten Hautpartien zur Anhaftung von Sand kommt. Dieses Problem wird umso größer, je mehr UV-Filter eine Zubereitung enthält. Zwar hat es in der Vergangenheit nicht an Versuchen gefehlt, sandabweisende Sonnenschutzmittel zu entwickeln, doch ist dieses Problem besonders bei Zubereitungen mit hohem Lichtschutzfaktor bis heute nicht endgültig zufriedenstellend gelöst.

Es war daher die Aufgabe der vorliegenden Erfindung, ein sandabweisendes Sonnenschutzmittel zu entwickeln. Insbesondere sollte ein Sonnenschutzmittel mit hohem Lichtschutzfaktor (SPF 50 und höher) entwickelt werden, welches besonders wenig sandanhaftend ist.

Kosmetische Sonnenschutzmittel haben neben der Klebrigkeit/Sandanhaftung darüber hinaus das Problem, dass eine Vielzahl von UV-Filtern in den Zubereitungen nicht besonders gut löslich ist. Insbesondere wenn Zubereitungen mit hohem Lichtschutzfaktor und hohem UV-Filtergehalt entwickelt werden, stellt die Löslichkeit von Triazinderivaten und 4-(tert.-Butyl)-4'-methoxydibenzoylmethan ein Problem für die Entwickler dar. Um dieses Problem zu lösen wurde in der Vergangenheit der flüssige UV-B-Filter Octocrylen als UV-Filter und Lösungsmittel verwendet.

Ein weiterer Nachteil des Standes der Technik besteht in dem Umstand, dass eine Reihe von weiteren Inhaltsstoffen in Sonnenschutzmitteln photolabil sind und/oder unter thermischer Belastung zerstört werden oder sich verflüchtigen. Die Photolabilität des UV-A-Filters und 4-(tert.-Butyl)-4'-methoxydibenzoylmethan stellt dabei eine besondere Herausforderung dar. Zur Photostabilisierung von 4-(tert.-Butyl)-4'-methoxydibenzoylmethan wird nach dem Stande der Technik ebenfalls bevorzugt Octocrylen verwendet.

Der Nachteil des Standes der Technik besteht nun in dem Umstand, dass der Einsatz von Octocrylen, trotz der Zulassung durch die Genehmigungsbehörden, nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass dieser UV-Filter möglicherweise hormonell wirksam sein könnte. Auch wenn, trotz des jahrzehntelangen weltweiten Einsatzes dieses UV-Filters in Sonnenschutzmitteln keine negativen Wirkungen am Menschen bekannt geworden sind, besteht bei den Verbrauchern der Wunsch, Zubereitungen mit derartigen Inhaltsstoffen zu meiden.

Es war daher die Aufgabe der vorliegenden Erfindung, die Nachteile des Standes der Technik zu beseitigen und ein sandabweisendes Sonnenschutzmittel mit hohem Lichtschutzfaktor zu zu entwickeln, bei dem die UV-Filter stabil gelöst und der photochemische Abbau von 4-(tert.-Butyl)-4'-methoxydibenzoylmethan unterdrückt wird. Idealerweise sollte die Aufgabe gelöst werden ohne dass Octocrylen als Lösungsmittel und Stabilisator eingesetzt wird.

Überraschend gelöst werden die Aufgaben durch ein kosmetisches Sonnenschutzmittel enthaltend eine UV-Filterkombination

a) 4-(tert.-Butyl)-4'-methoxydibenzoylmethan,
b) 2,4,6-Tris-[anilino-(p-carbo-2'-ethyl-1'-hexyloxy)]-1,3,5-triazin (INCI: Ethylhexyl Triazone),
c) 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine),
d) 2-Phenylbenzimidazol-5-sulfonsäuresalze,

wobei die Zubereitung ein oder mehrere Alkandiole aus der Gruppe der Verbindungen 1,2-Pentandiol, 1,2-Hexandiol,

1,2-Octandiol, 1,2-Decandiol, 2-Methyl-1,3-propandiol enthält, dadurch gekennzeichnet, dass die Zubereitung kein Titandioxid, Zinkoxid, 3-(4-Methylbenzyliden)-campher, 2-Hydroxy-4-methoxybenzophenon (INCI: Oxybenzon) und Ethylhexyl-2-cyano-3,3-diphenylacrylat (INCI: Octocrylen) enthält.

Zwar kennt der Stand der Technik die DE 1020122000074, US 2013/344015 und DE 19846771 sowie den Datenbankeintrag der Mintel-Datenbank GNPD mit der Record ID 1826191 "Protect & Tan Sun Milk SPF 20 Medium, doch konnten diese Schriften nicht den Weg zur vorliegenden Erfindung weisen.

Die erfindungsgemäßen Zubereitungen kommen mit einer überraschend geringen Gesamtmenge an UV-Filtern aus.

Die erfindungsgemäße Zubereitung kann, neben der beanspruchten UV-Filterkombination weitere UV-Filter enthalten. Diese können erfindungsgemäß vorteilhaft aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; Terephthalidendicampher-sulfonsäure; 4-(Dimethylamino)-benzoesäure(2-ethylhexyl)ester; 4-(Dimethylamino)-benzoesäureamylester; 4-Methoxybenzalmalon-säuredi(2-ethylhexyl)ester; 4-Methoxyzimtsäureisoamylester; 2-(4'-Diethylamino-2'-hydoxybenzoyl)-benzoesäurehexylester, Ethylhexylsalicylat, Homomenthylsalicylat, 2-Ethylhexyl-2-hydroxy-benzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamido-triazon (INCI: Diethylhexyl-Butamidotriazone); 2,4,6-Tribiphenyl-4-yl-1,3,5-triazin.

Es ist erfindungsgemäß vorteilhaft, wenn die erfindungsgemäße Zubereitung in Form einer Emulsion oder Dispersion, bevorzugt in Form einer Emulsion und besonders bevorzugt in Form einer O/W-Emulsion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate , Natriumstearylglutamat, enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Es ist erfindungsgemäß bevorzugt, wenn die Zubereitung Natriumstearylglutamat als Emulgator enthält.

Es ist ferner erfindungsgemäß vorteilhaft, wenn die Zubereitung Cetylalkohol, Stearylalkohol und/oder Glycerylstearat enthält.

Es ist erfindungsgemäß von Vorteil, wenn die erfindungsgemäße Zubereitung frei ist von Polyethylenglycol, Polyethylenglycolethern und Polyethylenglycolestern (sogenannten PEG-Derivaten).

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13

Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindugnsgemäß bevorzugt, wenn die Zubereitung Dibutyladipat, Dicaprylylcarbonat und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Es ist dabei erfindungsgemäß bevorzugt, wenn die Zubereitung Xanthangummi, quervernetztes Acrylat/C10-C30 Alkylacrylat Polymer und/oder Vinylpyrrolidon/Hexadecen-Copolymer enthält.

Ein Gehalt an Glycerin von mindestens 5 Gewicht-%, bezogen auf das Gesamtgewicht der Zubereitung, ist erfindungsgemäß besonders vorteilhaft.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Es ist erfindungsgemäß vorteilhaft, wenn die erfindungsgemäße Zubereitung Ethanol, Phenoxyethanol und/oder Ethylhexylglycerin enthält.

Erfindungsgemäß vorteilhafte Ausführungsformen der vorliegenden Erfindung sind dadurch gekennzeichnet dass die Zubereitung frei ist von Parabenen, Methylisothiazolinon, Chlormethylisothiazolinon und DMDM-Hydantoin.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im Wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP)

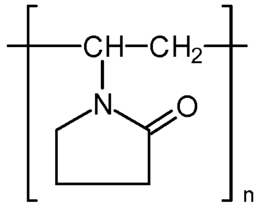

zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Erfindungsgemäß ist die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß ist auch die Verwendung der erfindungsgemäßen UV-Filterkombination zur Reduzierung der Sandanhaftung in kosmetischen Zubereitungen (insbesondere Sonnenschutzmitteln). Dabei ist insbesondere die Verwendung von 2-Phenylbenzimidazol-5-sulfonsäuresalzen sowie die Verwendung von 2,4,6-Tris-[anilino-(p-carbo-2'-ethyl-1'-hexyloxy)]-1,3,5-triazin (INCI: Ethylhexyl Triazone) zur Reduzierung der Sandanhaftung in kosmetischen Zubereitungen (insbesondere Sonnenschutzmitteln) erfindungsgemäß.

**Vergleichsversuch/Beispielrezeptur**

Es wurden die folgenden Rezepturen hergestellt und die Sandanheftung mit Hilfe der folgenden Methode bestimmt. Rezeptur 1 ist dabei eine erfindungsgemäße Ausführungsform:

| **Rezepturvergleich** | | | |
|---|---|---|---|
| | **Rezeptur 1** | **Rezeptur 2** | **Rezeptur 3** |
| **INCI** | **m [g]** | **m [g]** | **m [g]** |
| Glyceryl Stearate | 1,00 | 1,00 | 1,00 |
| Aqua | 52,72 | 54,02 | 55,72 |
| Dibutyl Adipate | 3,00 | 3,00 | 3,00 |
| Cetearyl Alcohol | 1,00 | 1,00 | 1,00 |
| Trisodium EDTA | 1,00 | 1,00 | 1,00 |
| C12-15 Alkyl Benzoate | 6,00 | 6,00 | 6,00 |
| Butyl Methoxydibenzoylmethane | 4,50 | 4,50 | 4,50 |
| Phenylbenzimidazole Sulfonic Acid | 1,00 | | 1,00 |
| VP/Hexadecene Copolymer | 0,50 | 0,50 | 0,50 |
| Glycerin + Aqua | 7,50 | 7,50 | 7,50 |
| Aqua + Sodium Hydroxide | 0,38 | 0,08 | 0,38 |
| Alcohol Denat. + Aqua | 4,00 | 4,00 | 4,00 |
| Xanthan Gum | 0,40 | 0,40 | 0,40 |
| Ethylhexyl Triazone | 3,00 | 3,00 | |
| Acrylates/C10-30 Alkyl Acrylate Crosspolymer | 0,10 | 0,10 | 0,10 |
| Silica Dimethyl Silylate | 0,50 | 0,50 | 0,50 |
| Ethylhexylglycerin | 0,50 | 0,50 | 0,50 |
| C18-38 Alkyl Hydroxystearoyl Stearate | 1,00 | 1,00 | 1,00 |
| Bis-Ethylhexyloxyphenol Methoxyphenyl Triazine | 3,50 | 3,50 | 3,50 |
| Butylene Glycol Dicaprylate/Dicaprate | 5,00 | 5,00 | 5,00 |
| Methylpropanediol | 3,00 | 3,00 | 3,00 |
| Sodium Stearoyl Glutamate | 0,40 | 0,40 | 0,40 |

(fortgesetzt)

| **Rezepturvergleich** | | | |
|---|---|---|---|
| | **Rezeptur 1** | **Rezeptur 2** | **Rezeptur 3** |
| **INCI** | **m [g]** | **m [g]** | **m [g]** |
| | | | |
| Summe | 100,00 | 100,00 | 100,00 |

***In-vitro* Sandanhaftung**

50 mg der Test-Emulsion wurden auf PMMA Schönberg Platten (5,0 x 5,0 cm) aufgetragen und gleichmäßig mit einem Fingerling auf der Platte verteilt. Anschließend wird die aufgetragene Beispielrezeptur für 15 min bei Raumtemperatur getrocknet. Danach wurde das Gewicht der getrockneten Platten mit einer Analysenwaage ermittelt. Anschließend wurden die Platten mit feinem Seesand (1.07711.1000 Seesand reinst, der Firma Merck KGaA) im Überschuss übergossen. Durch einmaliges Rutschen der Platten auf einer dafür vorgesehenen Rutschvorrichtung (siehe unten) wurde lose anhaftender Sand mit reproduzierbarer, einheitlicher Kraft entfernt.

Der daraufhin auf der Platte zurückbleibende anhaftende Sand wurde durch Auswiegen ermittelt. Die Sandanhaftung kann mit folgender Gleichung ermittelt werden:

$$\Delta\ (Anhaftung)\ [mg] = m\ (Platte\ mit\ Sand)[mg] - m\ (eingecremte\ Platte)\ [mg]$$

Die Rutschvorrichtung ist eine in Form eines Dreiecks aufgebaute Konstruktion, bei der die Breite der Rutsche 5 cm beträgt.

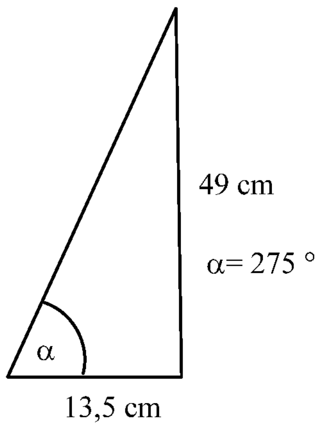


Die Versuche wurden je Rezeptur 10x wiederholt und der entsprechende Mittelwert gebildet.

| **Rezeptur 1** | | | | |
|---|---|---|---|---|
| Platte mit Emulsion | Platte mit Sand | Sand (anhaftend) | Mittelwert | Standardabweichung |
| 7,312 | 7,702 | 0,39 | | |
| 7,3 | 7,663 | 0,363 | | |
| 7,306 | 7,781 | 0,475 | | |
| 7,298 | 7,744 | 0,446 | | |
| 7,307 | 7,763 | 0,456 | | |
| 7,288 | 7,693 | 0,405 | | |
| 7,307 | 7,619 | 0,312 | | |

(fortgesetzt)

| Rezeptur 1 | | | | |
|---|---|---|---|---|
| Platte mit Emulsion | Platte mit Sand | Sand (anhaftend) | Mittelwert | Standardabweichung |
| 7,305 | 7,735 | 0,43 | | |
| 7,317 | 7,744 | 0,427 | | |
| 7,31 | 7,727 | 0,417 | 0,412 | 0,048 |
| Da die Platte 25 $cm^2$ groß ist : 16,48 $mg/cm^2$ bei 1,92 Standardabweichung | | | | |

| Rezeptur 2 | | | | |
|---|---|---|---|---|
| Platte mit Emulsion | Platte mit Sand | Sand (anhaftend) | Mittelwert | Standardabweichung |
| 7,312 | 7,826 | 0,514 | | |
| 7,302 | 7,76 | 0,48 | | |
| 7,317 | 7,793 | 0,476 | | |
| 7,299 | 7,669 | 0,37 | | |
| 7,322 | 7,622 | 0,3 | | |
| 7,294 | 7,75 | 0,456 | | |
| 7,308 | 7,749 | 0,441 | | |
| 7,307 | 7,725 | 0,418 | | |
| 7,321 | 7,666 | 0,345 | | |
| 7,3 | 7,7 | 0,4 | 0,42 | 0,067 |
| Da die Platte 25 $cm^2$ groß ist: 16,80 $mg/cm^2$ bei 2,68 Standardabweichung | | | | |

| Rezeptur 3 | | | | |
|---|---|---|---|---|
| Platte mit Emulsion | Platte mit Sand | Sand (anhaftend) | Mittelwert | Standardabweichung |
| 7,301 | 7,701 | 0,4 | | |
| 7,32 | 7,72 | 0,4 | | |
| 7,323 | 7,773 | 0,45 | | |
| 7,302 | 7,702 | 0,4 | | |
| 7,296 | 7,716 | 0,42 | | |
| 7,307 | 7,887 | 0,58 | | |
| 7,318 | 7,742 | 0,424 | | |
| 7,31 | 7,72 | 0,41 | | |
| 7,309 | 7,849 | 0,54 | | |
| 7,315 | 7,735 | 0,42 | 0,444 | 0,063 |
| Da die Platte 25 $cm^2$ groß ist: 17,76 $mg/cm^2$ bei 2,52 Standardabweichung | | | | |

**Fazit:** Die erfindungsgemäße UV-Filterkombination (Rezeptur 1) zeigt eine signifikant geringere Sandanhaftung als die Zubereitungen, bei denen keine 2-Phenylbenzimidazolsulfonsäure oder Ethylhexyl Triazone enthalten ist.

## Patentansprüche

1. Kosmetisches Sonnenschutzmittel enthaltend eine UV-Filterkombination

   a) 4-(tert.-Butyl)-4'-methoxydibenzoylmethan,
   b) 2,4,6-Tris-[anilino-(p-carbo-2'-ethyl-1'-hexyloxy)]-1,3,5-triazin (INCI: Ethylhexyl Triazone),
   c) 2,4-Bis-{[4-(2-ethyl-hexyloxy)-2-hydroxy]-pheny}-6-(4-methoxyphenyl)-1,3,5-triazin (INCI: Bis- Ethylhexyloxyphenol methoxyphenyl Triazine),
   d) 2-Phenylbenzimidazol-5-sulfonsäuresalze,

   wobei die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält, **dadurch gekennzeichnet, dass** die Zubereitung kein Titandioxid, Zinkoxid, 3-(4-Methylbenzyliden)-campher, 2-Hydroxy-4-methoxybenzophenon (INCI: Oxybenzon) und Ethylhexyl-2-cyano-3,3-diphenylacrylat (INCI: Octocrylen) enthält.

2. Kosmetische Zubereitung nach Anspruch 1, **dadurch gekennzeichnet, dass** die Zubereitung in Form einer O/W-Emulsion vorliegt.

3. Kosmetische Zubereitung nach einem der vorhergehenden Ansprüche, **dadurch gekennzeichnet, dass** die Zubereitung Natriumstearylglutamat als Emulgator enthält.

4. Kosmetische Zubereitung nach einem der vorhergehenden Ansprüche, **dadurch gekennzeichnet, dass** die Zubereitung Silica Dimethyl Silylate enthält.

5. Kosmetische Zubereitung nach einem der vorhergehenden Ansprüche, **dadurch gekennzeichnet, dass** die Zubereitung Dibutyladipat, Dicaprylylcarbonat und/oder C12-C15 Alkylbenzoat enthält.

6. Kosmetische Zubereitung nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

7. Kosmetische Zubereitung nach einem der vorhergehenden Ansprüche, **dadurch gekennzeichnet, dass** die Zubereitung Ethanol, Phenoxyethanol und/oder Ethylhexylglycerin enthält.

8. Kosmetische Zubereitung nach einem der vorhergehenden Ansprüche, **dadurch gekennzeichnet, dass** die Zubereitung Xanthangummi, quervernetztes Acrylat/C10-C30 Alkylacrylat Polymer und/oder Vinylpyrrolidon/Hexadecen-Copolymer enthält.

9. Kosmetische Zubereitung nach einem der vorhergehenden Ansprüche, **dadurch gekennzeichnet, dass** die Zubereitung Cetylalkohol, Stearylalkohol und/oder Glycerylstearat enthält.

10. Kosmetische Zubereitung nach einem der vorhergehenden Ansprüchen, **dadurch gekennzeichnet dass** die Zubereitung frei ist von Parabenen, Methylisothiazolinon, Chlormethylisothiazolinon und DMDM-Hydantoin.

11. Kosmetische Zubereitung nach einem der vorhergehenden Ansprüche, **dadurch gekennzeichnet, dass** die Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

**Claims**

1. Cosmetic sunscreen composition comprising a UV filter combination of

a) 4-(tert-butyl)-4'-methoxydibenzoylmethane,
b) 2,4,6-tris[anilino(p-carbo-2'-ethyl-1'-hexyloxy)]-1,3,5-triazine (INCI: Ethylhexyl Triazone),
c) 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine),
d) 2-phenylbenzimidazole-5-sulfonic acid salts,

wherein the preparation comprises one or more alkanediols from the group of the compounds 1,2-pentanediol, 1,2-hexanediol, 1,2-octanediol, 1,2-decanediol, and 2-methyl-1,3-propanediol, **characterized in that** the preparation contains no titanium oxide, zinc oxide, 3-(4-methylbenzylidene)camphor, 2-hydroxy-4-methoxybenzophenone (INCI: Oxybenzone), and ethylhexyl 2-cyano-3,3-diphenylacrylate (INCI: Octocrylene).

2. Cosmetic preparation according to Claim 1, **characterized in that** the preparation is in the form of an O/W emulsion.

3. Cosmetic preparation according to any of the preceding claims, **characterized in that** the preparation comprises sodium stearylglutamate as emulsifier.

4. Cosmetic preparation according to any of the preceding claims, **characterized in that** the preparation comprises Silica Dimethyl Silylate.

5. Cosmetic preparation according to any of the preceding claims, **characterized in that** the preparation comprises dibutyl adipate, dicaprylyl carbonate and/or C12-C15 alkylbenzoate.

6. Cosmetic preparation according to any of the preceding claims, **characterized in that** the preparation comprises one or more compounds selected from the group of the compounds alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, tocopheryl acetate, dihydroxyacetone; 8-hexadecene-1,16-dicarboxylic acid, glycerylglycose, (2-hydroxyethyl)urea, vitamin E and/or derivatives thereof, hyaluronic acid and/or salts thereof, and/or licochalcone A.

7. Cosmetic preparation according to any of the preceding claims, **characterized in that** the preparation comprises ethanol, phenoxyethanol and/or ethylhexylglycerol.

8. Cosmetic preparation according to any of the preceding claims, **characterized in that** the preparation comprises xanthan gum, crosslinked acrylate/C10-C30 alkyl acrylate polymer, and/or vinylpyrrolidone/hexadecene copolymer.

9. Cosmetic preparation according to any of the preceding claims, **characterized in that** the preparation comprises cetyl alcohol, stearyl alcohol and/or glyceryl stearate.

10. Cosmetic preparation according to any of the preceding claims, **characterized in that** the preparation is free from parabens, methylisothiazolinone, chloromethylisothiazolinone, and DMDM-hydantoin.

11. Cosmetic preparation according to any of the preceding claims, **characterized in that** the preparation comprises one or more fragrances selected from the group of the compounds limonene, citral, linalool, alpha-isomethylionone, geraniol, citronellol, 2-isobutyl-4-hydroxy-4-methyltetrahydropyran, 2-tert-pentylcyclohexyl acetate, 3-methyl-5-phenyl-1-pentanol, 7-acetyl-1,1,3,4,4,6-hexamethyltetralin, adipic diester, alpha-amylcinnamaldehyde, alpha-methylionone, amyl C, butylphenylmethylpropional cinnamal, amyl salicylate, amylcinnamyl alcohol, anisyl alcohol, benzoin, benzyl alcohol, benzyl benzoate, benzyl cinnamate, benzyl salicylate, bergamot oil, bitter orange oil, butylphenylmethylpropional, cardamom oil, cedrol, cinnamal, cinnamyl alcohol, citronellyl methylcrotonate, citrus oil, coumarin, diethyl succinate, ethyllinalool, eugenol, Evernia furfuracea extract, Evernia prunastri extract, farnesol, guaiacwood oil, hexylcinnamal, hexyl salicylate, hydroxycitronellal, lavender oil, lemon oil, linalyl acetate, mandarin oil, menthyl PCA, methylheptenone, nutmeg oil, rosemary oil, sweet orange oil, terpineol, tonkabean oil, triethyl citrate and/or vanillin.

## Revendications

1. Agent cosmétique de protection solaire, contenant une combinaison de filtres UV :

   a) le 4-(tert.-butyl)-4'-méthoxydibenzoylméthane,
   b) la 2,4,6-tris-[anilino-(p-carbo-2'-éthyl-1'-hexyloxy)]-1,3,5-triazine (INCI : Ethylhexyl Triazone),
   c) la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine),
   d) les sels de l'acide 2-phénylbenzimidazole-5-sulfonique, la préparation contenant un ou plusieurs alcanediols du groupe de composés constitué par le 1,2-pentanediol, le 1,2-hexanediol, le 1,2-octanediol, le 1,2-décanediol, le 2-méthyl-1,3-propanediol, **caractérisé en ce que** la préparation ne contient pas de dioxyde de titane, d'oxyde de zinc, de 3-(4-méthylbenzylidène)-camphre, de 2-hydroxy-4-méthoxybenzophénone (INCI : Oxybenzon) et d'acrylate d'éthylhexyl-2-cyano-3,3-diphényle (INCI : Octocrylen).

2. Préparation cosmétique selon la revendication 1, **caractérisée en ce que** la préparation se présente sous la forme d'une émulsion H/E.

3. Préparation cosmétique selon l'une quelconque des revendications précédentes, **caractérisée en ce que** la préparation contient du glutamate de stéaryle sodique en tant qu'émulsifiant.

4. Préparation cosmétique selon l'une quelconque des revendications précédentes, **caractérisée en ce que** la préparation contient du Silica Dimethyl Silylate.

5. Préparation cosmétique selon l'une quelconque des revendications précédentes, **caractérisée en ce que** la préparation contient de l'adipate de dibutyle, du carbonate de dicaprylyle et/ou du benzoate d'alkyle en C12-C15.

6. Préparation cosmétique selon l'une quelconque des revendications précédentes, **caractérisée en ce que** la préparation contient un ou plusieurs composés choisis dans le groupe de composés constitué par l'acide alpha-liponique, l'acide folique, le phytoène, la D-biotine, la coenzyme Q10, l'alpha-glucosylrutine, la carnitine, la carnosine, les isoflavonoïdes naturels et/ou synthétiques, les flavonoïdes, la créatine, la créatinine, la taurine, la β-alanine, l'acétate de tocophéryle, la dihydroxyacétone, l'acide 8-hexadécène-1,16-dicarboxylique, le glycérylglucose, la (2-hydroxyéthyl)urée, la vitamine E et/ou ses dérivés, l'acide hyaluronique et/ou ses sels et/ou la licochalcone A.

7. Préparation cosmétique selon l'une quelconque des revendications précédentes, **caractérisée en ce que** la préparation contient de l'éthanol, du phénoxyéthanol et/ou de l'éthylhexylglycérine.

8. Préparation cosmétique selon l'une quelconque des revendications précédentes, **caractérisée en ce que** la préparation contient de la gomme xanthane, un polymère d'acrylate/acrylate d'alkyle en C10-C30 réticulé et/ou un copolymère de vinylpyrrolidone/hexadécène.

9. Préparation cosmétique selon l'une quelconque des revendications précédentes, **caractérisée en ce que** la préparation contient de l'alcool cétylique, de l'alcool stéarylique et/ou du stéarate de glycéryle.

10. Préparation cosmétique selon l'une quelconque des revendications précédentes, **caractérisée en ce que** la préparation est exempte de parabènes, de méthylisothiazolinone, de chlorométhylisothiazolinone et de DMDM-hydantoïne.

11. Préparation cosmétique selon l'une quelconque des revendications précédentes, **caractérisée en ce que** la préparation contient un ou plusieurs parfums choisis dans le groupe de composés constitué par le limonène, le citral, le linalool, l'alpha-isométhylionone, le géraniol, le citronellol, le 2-isobutyl-4-hydroxy-4-méthyltétrahydropyrane, l'acétate de 2-tert-pentylcyclohexyle, le 3-méthyl-5-phényl-1-pentanol, la 7-acétyl-1,1,3,4,4,6-hexaméthyltétraline, le diester de l'acide adipique, l'alpha-amylcinnamaldéhyde, l'alpha-méthylionone, l'amyl C butylphénylméthylpropionalcinnamal, le salicylate d'amyle, l'alcool amylcinnamylique, l'alcool anisique, la benzoïne, l'alcool benzylique, le benzoate de benzyle, le cinnamate de benzyle, le salicylate de benzyle, l'huile de bergamote, l'huile d'orange amère, le butylphénylméthylpropioal, l'huile de cardamome, le cédrol, le cinnamal, l'alcool cinnamylique, le crotonate de citronellylméthyle, l'huile de citron, la coumarine, le succinate de diéthyle, l'éthyllinalool, l'eugénol, l'extrait d'Evernia Furfuracea, l'extrait d'Evernia Prunastri, le farnésol, l'huile de bois de gaïac, l'hexylcinnamal, le salicylate d'hexyle, l'hydroxycitronellal, l'huile de lavande, l'huile de citron, l'acétate de linayle, l'huile de mandarine, le menthyl PCA,

la méthylhepténone, l'huile de noix de muscade, l'huile de romarin, l'huile d'orange douce, le terpinéol, l'huile de fève tonka, le citrate de triéthyle et/ou la vanilline.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 1020122000074
- US 2013344015 A
- DE 19846771


### In der Beschreibung aufgeführte Nicht-Patentliteratur


- *CHEMICAL ABSTRACTS,* 178463-23-5
- *CHEMICAL ABSTRACTS,* 7631-86-9
- *CHEMICAL ABSTRACTS,* 646054-62-8
- *CHEMICAL ABSTRACTS,* 403517-45-3